# FASCICULE DE BREVET EUROPEEN

(11) **EP 2 643 011 B1**
(45) Date de publication et mention de la délivrance du brevet: **13.07.2016**
(21) Numéro de dépôt: 11797266.1
(22) Date de dépôt: 23.11.2011
(51) Int. Cl.: A61K 38/17, A61P 19/08

(54) **ISOFORME D'HLA-G POUR UTILISATION DANS LE TRAITEMENT D'UNE MALADIE ASSOCIÉE À UNE RÉSORPTION OSSEUSE**
ISOFORM VON HLA-G FÜR DIE VERWENDUNG IN DER BEHANDLUNG VON ERKRANKUNGEN, DIE MIT KNOCHENRESORPTION VERBUNDEN SIND
HLA-G ISOFORM FOR USE IN THE TREATMENT OF A DISEASE ASSOCIATED WITH OSSEOUS RESORPTION

(30) Priorité: 23.11.2010 FR 1059653
(43) Date de publication de la demande: 02.10.2013
(73) Titulaire: Etablissement Français du Sang, 93218 La Plaine Saint Denis Cedex (FR); Commissariat à l'Énergie Atomique et aux Énergies Alternatives, 75015 Paris (FR)
(72) Inventeur: DESCHASEAUX, Frédéric, F-37000Tours (FR); SENSEBE, Luc, F-37300 Joue Les Tours (FR); ROUAS-FREISS, Nathalie, F-75010 Paris (FR)
(74) Mandataire: Gevers & Orès
(86) Numéro de dépôt international: PCT/IB2011/055255
(87) Numéro de publication internationale: WO 2012/070003

(56) Documents cités:
- WO-A1-2011/061726
- WO-A2-2008/051568
- FR-A1- 2 760 023
- ONGARO A ET AL: "Human leukocyte antigen-G molecules are constitutively expressed by synovial fibroblasts and upmodulated in osteoarthritis", HUMAN IMMUNOLOGY, NEW YORK, NY, US, vol. 71, no. 4, 1 avril 2010 (2010-04-01), pages 342-350, XP026966132, ISSN: 0198-8859 [extrait le 2010-02-06]
- ZOHAIR SELMANI ET AL: "Human Leukocyte Antigen-G5 Secretion by Human Mesenchymal Stem Cells Is Required to Suppress T Lymphocyte and Natural Killer Function and to Induce CD4 + CD25 high FOXP3 + Regulatory T Cells", STEM CELLS, vol. 26, no. 1, 1 janvier 2008 (2008-01-01), pages 212-222, XP055002928, ISSN: 1066-5099, DOI: 10.1634/stemcells.2007-0554 cité dans la demande
- J. LORENZO ET AL: "Osteoimmunology: Interactions of the Bone and Immune System", ENDOCRINE REVIEWS, vol. 29, no. 4, 1 janvier 2008 (2008-01-01), pages 403-440, XP055002942, ISSN: 0163-769X, DOI: 10.1210/er.2007-0038 cité dans la demande
- UCCELLI A ET AL: "Mesenchymal stem cells in health and disease", NATURE REVIEWS. IMMUNOLOGY, NATURE PUBLISHING GROUP, GB, vol. 8, no. 9, 1 septembre 2008 (2008-09-01), pages 726-736, XP007913720, ISSN: 1474-1733, DOI: DOI:10.1038/NRI2395 [extrait le 2008-08-18]
- F. DESCHASEAUX ET AL: "The human non-classical type I CMH HLA-G proteins show restricted expression by osteoblastic lineage in normal and tumoral conditions", BONE, vol. 48, no. Suppl. 2, 7 mai 2011 (2011-05-07), page S109, XP55017991, ISSN: 8756-3282, DOI: 10.1016/j.bone.2011.03.179

## Description

La présente invention est relative à une nouvelle utilisation des isoformes d'HLA-G comme agents limitant la résorption osseuse, pour le traitement, par exemple, de l'ostéoporose.

Les antigènes du complexe majeur d'histocompatibilité (CMH) se divisent en plusieurs classes : les antigènes de classe I (HLA-A, -B et -C) qui présentent 3 domaines globulaires (α1, α2 et α3), et dont le domaine α3 est associé à la β2 microglobuline, les antigènes de classe II (HLA-DP, -DQ et -DR) et les antigènes de classe III (complément). Les antigènes de classe I comprennent, outre les antigènes précités, d'autres antigènes dits antigènes de classe I non classiques (classe Ib) et notamment les antigènes HLA-E, HLA-F et HLA-G (Carosella *et al.,* 2008a).

La séquence nucléotidique du gène *HLA-G* (dénommé aussi gène *HLA-6.0*) a été décrite par Geraghty *et al.* en 1987 : il comprend 4396 paires de bases et présente une organisation en intron/exon homologue à celle des gènes *HLA-A, -B* et -*C*. Ce gène *HLA-G* comprend 8 exons, 7 introns et une extrémité 3' non traduite. Il diffère des autres gènes codant pour les antigènes de classe I en ce que le codon de terminaison de la traduction en phase est localisé au niveau du deuxième codon de l'exon 6 ; en conséquence, la région cytoplasmique de la protéine codée par ce gène est plus courte que celle des régions cytoplasmiques des protéines HLA-A, -B et -C. Ishitani et Geraghty (1992) ont montré que le transcrit primaire du gène *HLA-G* peut être épissé de plusieurs manières et produit au moins 3 ARNm matures distincts : le transcrit primaire d'*HLA-G* fournit une copie complète G1 de 1200 pb, un fragment de 900 pb (G2) et un fragment de 600 pb (G3). Le transcrit G1 ne comprend pas l'exon 7 et correspond à la séquence décrite par Ellis en 1990, c'est-à-dire qu'il code une protéine qui comprend une séquence signal, 3 domaines externes, une région transmembranaire et une séquence cytoplasmique. L'ARNm G2 ne comprend pas l'exon 3, c'est-à-dire qu'il code une protéine dans laquelle les domaines α1 et α3 sont directement joints. L'ARNm G3 ne contient ni l'exon 3, ni l'exon 4, c'est-à-dire qu'il code une protéine dans laquelle le domaine α1 et la séquence transmembranaire sont directement joints. L'épissage qui prévaut pour l'obtention de l'antigène G2 entraîne la jonction d'une adénine (A) (provenant du domaine codant α1) avec une séquence adénine-cytosine (AC) (issue du domaine codant α3), ce qui entraîne la création d'un codon AAC (asparagine) à la place du codon GAC (acide aspartique), présent en position 5' de la séquence codant le domaine α3 dans HLA-G1. L'épissage généré pour l'obtention d'HLA-G3 n'entraîne pas la formation d'un nouveau codon dans la zone d' épissage.

Il a également été montré l'existence d'autres formes épissées d'ARNm d'HLA-G : le transcrit G4, qui n'inclut pas l'exon 4 ; le transcrit G5 qui inclut l'intron 4 entre l'exon 4 et 5 provoquant ainsi une modification du cadre de lecture lors de la traduction de ce transcrit et en particulier l'apparition d'un codon stop après l'acide aminé 21 de l'intron 4 ; le transcrit G6 possédant l'intron 4 mais ayant perdu l'exon 3 et enfin le transcrit G7 qui inclut l'intron 2 provoquant ainsi une modification du cadre de lecture lors de la traduction de ce transcrit et l'apparition d'un codon stop après l'acide aminé 2 de l'intron 2 (Kirszenbaum *et al.,* 1994 et 1995 ; Moreau *et al.,* 1995 ; Demande de Brevet EP 0 677 582).

Il existe donc au moins 7 ARNm *HLA-G* différents qui codent 7 isoformes d'HLA-G dont 4 membranaires (HLA-G1, -G2, -G3 et -G4) et 3 solubles (HLA-G5, -G6 et -G7) (Carosella *et al.,* 2008b).

HLA-G a été initialement décrite comme spécifiquement exprimée par le trophoblaste au niveau de la barrière placentaire à l'interface foeto-maternelle (Carosella *et al.,* 2008a). Elle a aussi été récemment détectée au niveau du thymus, de la cornée, des précurseurs endothéliaux, dans les cellules souches mésenchymateuses (dénommées également cellules stromales de moelle osseuse ou cellules stromales multipotentes de moelle osseuse, et notées MSC ou CSM) et dans les ostéoblastes normaux ou tumoraux (Deschaseaux *et al.,* 2009a). Néanmoins, les ARNm *HLA-G* sont détectés dans quasiment toutes les cellules de l'organisme à un taux basal qui peut être amplifié et dont la traduction en protéine est induite sous l'effet d'agents déméthylants de l'ADN, de certaines cytokines telles que l'IFN (Interferon), de facteurs de stress ou d'hypoxie (Carosella *et al.,* 2008a). Ainsi, dans des conditions particulières telles que la greffe d'un tissu, le développement de certaines tumeurs ou une réponse inflammatoire, la protéine HLA-G pourra être exprimée dans des tissus qui ne l'expriment pas en condition normale. Dans le sang, on retrouve aussi bien les isoformes membranaires détachées des membranes telles que HLA-G1 (nommées alors HLA-G1s pour « HLA-G1 shedding ») que les autres isoformes solubles.

HLA-G se distingue des autres molécules HLA de classe I dans la mesure où :
- son expression est restreinte à certains tissus,
- elle présente très peu de polymorphisme (44 allèles codant pour 15 variants protéiques), ceci étant dû à de nombreuses mutations silencieuses, et
- elle induit une tolérance immunitaire.

Cette tolérance immunitaire peut s'expliquer par plusieurs mécanismes : HLA-G inhibe l'activation des cellules NK via sa liaison aux récepteurs ILT2 (CD85j) et KIR2DL4 (CD158d). ILT2 est également exprimé par les cellules monocytaires, les cellules dendritiques, les lymphocytes B et T et les macrophages (Carosella *et al.,* 2008a ; Rouas-Freiss *et al.,* 2007). En se liant au récepteur ILT2, HLA-G est capable d'inhiber la prolifération des lymphocytes T (CD4⁺ et CD8⁺) ainsi que leur potentiel cytotoxique et peut induire la formation de lymphocytes suppresseurs ou régulateurs (Barrow et Trowsdale, 2006 ; Le Maoult *et al.,* 2005 ; Naji *et al.* 2007a). HLA-G se lie également à ILT4 (CD85d) exprimé sur les cellules de type myéloïde, à savoir, les monocytes et les cellules dendritiques. La liaison d'HLA-G à ILT2 et/ou ILT4 entraîne l'inhibition de la maturation des cellules dendritiques et la génération de cellules dendritiques de type tolérogène. Les récepteurs ILT2 et ILT4 sont des molécules transmembranaires possédant un fragment cytoplasmique long contenant des motifs ITIM ou « *Immunoreceptor Tyrosine-based Inhibitory Motifs* ». Ils induisent l'inhibition de l'activation cellulaire par le recrutement de phosphatases SHP-1 ou SHP-2 à travers une hyperphosphorylation de ces protéines (Barrow et Trowsdale, 2006).

Le tissu osseux constituant le squelette est constamment renouvelé au cours de la vie de l'individu. Ce mécanisme de renouvellement est appelé le remodelage osseux. Il s'effectue à travers une balance finement régulée entre la formation osseuse et la résorption osseuse. Alors que la formation osseuse implique la synthèse et le dépôt de matrice osseuse (MO) par les ostéoblastes, la résorption osseuse se fait elle, par la dégradation de la MO par les ostéoclastes (Cohen, 2006).

Les ostéoblastes sont issus de cellules souches mésenchymateuses. Ce sont des cellules mononucléées présentes dans le tissu osseux en croissance et bordant l'os. Elles expriment, de façon caractéristique, du collagène I (colla1), la phosphatase alcaline non spécifique (ALPL), le récepteur de type 1 de l'hormone parathyroïdienne (PTH-R1), l'ostéonectine (SPARC), l'ostéocalcine et le facteur de transcription ostérix. Les ostéoblastes peuvent ensuite devenir des ostéocytes enchâssés dans la MO assurant ainsi son maintien (Deschaseaux *et al.,* 2009b).

Les ostéoclastes sont des cellules multinucléées exprimant la protéine TRAP (« *Tartrate Resistant Phosphatase* »). Elles sont issues de cellules de la lignée monocytaire sous l'action du facteur soluble RANKL (« *Receptor Activator of NF-kB Ligand* » ou TNFSF11) qui peut être secrété par les ostéoblastes eux-mêmes mais aussi par des cellules de type lymphocytaire (Duplomb *et al.,* 2007).

La dérégulation du remodelage osseux entraine un certain nombre de pathologies chez l'Homme (Cohen, 2006). Un excès de formation osseuse peut se traduire par de l'ostéopétrose alors qu'un excès de résorption osseuse est la cause de l'ostéoporose (maladie touchant 19 millions d'européens dont 1 femme sur 3 et 1 homme sur 8 ; 1,5 millions de personnes en Europe souffrent de fracture due à l'ostéoporose. Données provenant de l'association Health First Europe) ou plus rarement de la maladie de Paget ou des tumeurs ostéolytiques (métastases osseuses du cancer du sein ou de la prostate).

Au regard de ce qui précède, il existe donc un besoin de disposer de molécules pour traiter ou prévenir des disfonctionnements du remodelage osseux, notamment pour traiter l'ostéoporose.

Les principales molécules utilisées pour traiter l'ostéoporose sont soit des molécules chimiques de synthèse appartenant à la famille des biphosphonates qui inhibent l'activité des ostéoclastes (e.g., l'étidronate, le clodronate, le pamidronate, l'ibandronate, l'alendronate et le tiludronate ; Deschaseaux *et al.,* 2009a), soit des anticorps humanisés (e.g., le denosumab) mimant l'action de l'ostéoprotégérine qui est un inhibiteur compétitif du RANKL (Deschaseaux *et al.,* 2009a).

RANKL, également appelé « *TNF-related activation-induced cytokine* » (TRANCE), « *osteoprotegerin ligand* » (OPGL) ou « *osteoclast differentiation factor* » (ODF), est une cytokine qui intervient dans le métabolisme osseux en induisant la génération d'ostéoclastes et en les activant (Duplomb *et al.,* 2007).

L'ostéoprotégérine (OPG) est un récepteur soluble, appartenant à la famille de récepteurs des TNF (« *tumor necrosis factor* »), auquel se lie RANKL. La liaison de l'OPG à RANKL bloque l'interaction de ce dernier avec le récepteur RANK exprimé par les ostéoclastes et les monocytes (ladite interaction étant responsable de la différenciation et de l'activation des ostéoclastes). RANKL et OPG jouent donc un rôle clé dans la différenciation et l'activation des ostéoclastes (Cohen, 2006 ; Duplomb *et al.,* 2007 ; Lorenzo *et al.,* 2008). WO 2008051568 decrit l'utilisation des cellules souches placentaires positives pour l'HLA-G pour le traitement des défauts osseux.

Les Inventeurs ont montré, de manière inattendue, que différentes isoformes d'HLA-G isolées, en particulier les isoformes solubles, sont capables d'inhiber *in vitro* non seulement la formation des ostéoclastes dont la fonction biologique est de résorber la matrice osseuse, mais aussi l'ostéoclastogenèse. Les isoformes d'HLA-G peuvent donc être utilisées en tant qu'agents anti-résorption osseuse.

La présente invention a en conséquence pour objet une isoforme d'HLA-G isolée pour utilisation comme médicament pour le traitement ou la prévention d'une maladie dans laquelle on observe une résorption osseuse.

Parmi les maladies dans lesquelles on observe une résorption osseuse concernées, on peut citer l'ostéoporose, l'ostéopénie, les fractures osseuses, la maladie de Paget et les tumeurs ostéolytiques.

On entend par « isoforme d'HLA-G » une isoforme d'HLA-G choisie parmi les isoformes d'HLA-G membranaires (i.e., HLA-G1, HLA-G2, HLA-G3 et HLA-G4) et solubles (*i.e.,* HLA-G5, HLA-G6 et HLA-G7).

On entend par « isolée », une isoforme d'HLA-G qui est séparée d'un composé ou d'un milieu avec lequel elle est naturellement associée, par exemple une membrane cellulaire ou le cytoplasme. Ainsi, lorsqu'une isoforme d'HLA-G membranaire est utilisée pour la réalisation de la présente invention, cela signifie que cette isoforme est séparée (isolée) de la membrane cellulaire, telle que par exemple de la membrane cellulaire d'une cellule souche, en particulier une cellule souche placentaire.

A titre d'exemple, les isoformes membranaires peuvent être avantageusement exprimées dans des cellules eucaryotes, conformément au procédé décrit dans la Demande Internationale PCT WO 98/37098, puis solubilisées par traitement de la membrane (agent décapant, tel que la papaïne) et purification, par exemple sur colonne d'immunoaffinité avec des anticorps spécifiques.

Selon un mode de réalisation préféré de la présente invention, ladite isoforme d'HLA-G est choisie dans le groupe constitué par HLA-G1 et HLA-G5, de préférence HLA-G5.

Conformément à l'invention, l'isoforme d'HLA-G mise en oeuvre se présente :
- soit sous une forme libre (ou monomérique), qui peut éventuellement former des dimères en solution,
- soit sous une forme multimérique, notamment sous une forme agrégée sur des billes (tel que décrit par Naji *et al.,* 2007a), afin que la molécule d'HLA-G se présente sous une forme de multimères, décrits comme étant la conformation fonctionnellement optimale de la molécule HLA-G. En effet, les dimères d'HLA-G ont été décrits comme présentant une affinité considérablement augmentée pour leurs récepteurs comparativement aux monomères.

L'utilisation d'une isoforme d'HLA-G dans le traitement d'une maladie dans laquelle on observe une résorption osseuse peut constituer un traitement alternatif ou complémentaire en association avec les traitements habituellement mis en oeuvre.

La présente invention a également pour objet une composition pharmaceutique comprenant une isoforme d'HLA-G isolée telle que définie ci-dessus et au moins un véhicule pharmaceutiquement acceptable pour le traitement ou la prévention d'une maladie dans laquelle on observe une résorption osseuse telle que définie ci-dessus.

Selon un mode de réalisation préféré de ladite composition pharmaceutique, elle ne contient pas de cellules souches, en particulier de cellules souches placentaires.

Selon un mode de réalisation de ladite composition, ledit véhicule pharmaceutiquement acceptable est adapté à l'administration parentérale.

L'administration peut être par exemple intraveineuse, intramusculaire ou sous-cutanée.

Selon un autre mode de réalisation avantageux de ladite composition, ledit véhicule pharmaceutiquement acceptable est adapté à l'administration orale.

Selon un autre mode de réalisation avantageux de ladite composition, ledit véhicule pharmaceutiquement acceptable est adapté à l'administration par inhalation.

Des solutions ou des suspensions utilisées pour l'application sous cutanée incluent typiquement l'un ou plusieurs des composés suivants : un diluant stérile, tel que l'eau, pour préparation injectable, une solution saline physiologique, isotonique et tamponnée, des huiles, des polyéthylène glycols, la glycérine, le polypropylène glycol ou d'autres solvants synthétiques ; des agents antibactériens tels que l'alcool benzylique ou les méthylparabènes ; des antioxydants tels que l'acide ascorbique ou le bisulfite de sodium ; des agents chélatants tels que l'acide éthylènediamine tétraacétique ; des tampons tels que des acétates, des citrates ou des phosphates ; et des agents pour l'ajustement de la tonicité tels que le chlorure de sodium ou le dextrose. Le pH peut être ajusté avec des acides ou des bases tels que l'acide chlorhydrique ou l'hydroxyde de sodium.

De telles préparations peuvent se présenter sous la forme d'ampoules, de seringues à jeter ou de flacons multidoses en verre ou en plastique.

Les compositions pharmaceutiques adaptées à l'injection incluent des solutions aqueuses stériles, des dispersions ou des poudres stériles pour une préparation extemporanée de solutions ou de dispersions injectables stériles.

Pour l'administration en intraveineuse, les véhicules préférés incluent les solutions salines physiologiques, l'eau bactériostatique, le Cremophor ELTM (BASF, Parsippany, NJ) ou le tampon PBS. Dans tous les cas, la composition doit être stérile et fluide. Elle doit être stable dans les conditions de préparation et de stockage et doit comprendre des conservateurs contre l'action contaminante des microorganismes tels que les bactéries ou les champignons.

A titre d'exemple, le véhicule peut être un solvant ou un milieu de dispersion contenant par exemple de l'eau, de l'éthanol, un polyol (par exemple le glycérol, le propylène glycol ou un polyéthylène glycol liquide) et des mélanges de ces composés.

La fluidité correcte peut être maintenue par exemple en utilisant de la lécithine, ou en utilisant des agents surfactifs. La prévention de l'action des microorganismes peut être obtenue par l'administration de différents agents antibactériens et antifongiques, par exemple les parabènes, le chlorobutanol, le phénol, l'acide ascorbique et le thimérosal. Lesdites compositions peuvent également inclure des agents isotoniques, par exemple des sucres ou des polyalcools tels que le mannitol, le sorbitol ou le chlorure de sodium.

Les actions prolongées des compositions injectables peuvent être obtenues en ajoutant dans la formulation du monostéarate d'aluminium ou de la gélatine.

Outre les dispositions qui précèdent, l'invention comprend d'autres dispositions, qui ressortiront de la description qui va suivre, qui se réfère à un exemple montrant l'inhibition *in vitro* des ostéoclastes par les isoformes d'HLA-G, ainsi qu'aux dessins annexés, dans lesquels :

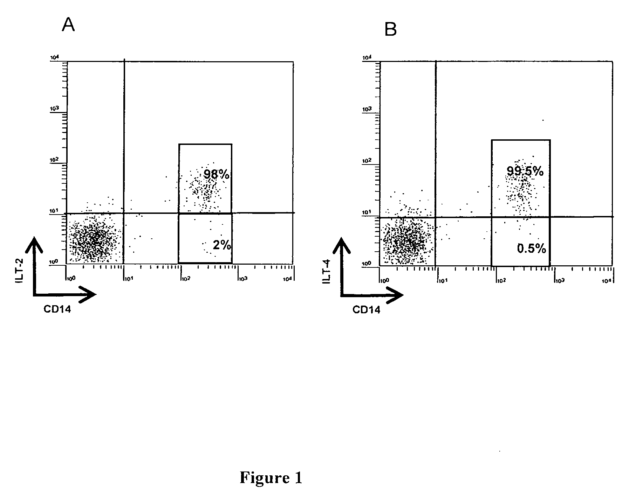

- la **Figure 1** est un graphique montrant que seules les cellules monocytaires CD14^{pos} expriment les récepteurs ILT2 (A) et ILT4 (**B**) dans des co-cultures avec des CSM CD14^{neg}. Des CSM de moelles osseuses d'origine humaine et des cellules monocytaires de sang périphérique humain ont été co-cultivées. Après environ 6 jours, les cellules ont été récupérées et marquées par des anticorps anti-CD14, anti-ILT2 ou anti-ILT4. Elles ont ensuite été analysées par cytométrie en flux.

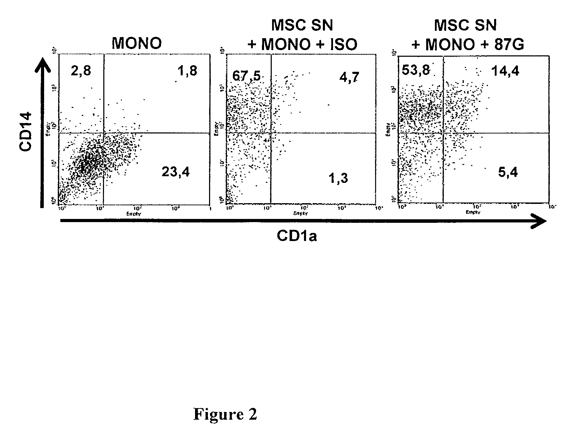

- la **Figure 2** est un graphique montrant l'action d'HLA-G issues de CSM dans l'inhibition de la différenciation des cellules dendritiques à partir de monocytes de sang périphérique. Des cellules monocytaires d'origine humaine ont été induites à se différencier en cellules dendritiques. Des fractions de ces cellules ont été cultivées avec des surnageants issus de cultures de CSM (MSC SN) auxquels il a été ajouté l'anticorps bloquants 87G (MSC SN + MONO + 87G) ou un anticorps contrôle de même isotype que 87G (MSC SN + MONO + ISO). Une autre fraction de cellules monocytaires a été induite à se différencier sans ajout de surnageant ni d'anticorps (MONO). Après 6 jours de cultures, les cellules ont été récoltées et analysées par cytométrie en flux pour l'expression de marqueurs monocytaires (CD14) et dendritiques (CD1a). 23% des cellules monocytaires se sont différenciées en cellules dendritiques (CD14^{neg}/CD1a^{pos}) dans les conditions contrôles (MONO). La plupart des cellules cultivées avec du surnageant de CSM et un anticorps contrôle sont CD14^{pos}/CD1a^{neg} alors qu'une fraction des cellules CD14^{pos} co-expriment le CD1a dans les conditions comprenant l'anticorps anti-HLA-G bloquant 87G (MSC SN + MONO + 87G). La présence d'HLA-G dans les surnageants de MSC a donc inhibé la différenciation des monocytes en cellules dendritiques. Le blocage d'HLA-G par un anticorps spécifique a permis de restaurer la différentiation des monocytes en une population intermédiaire CD14^{pos} CD1a^{pos}.

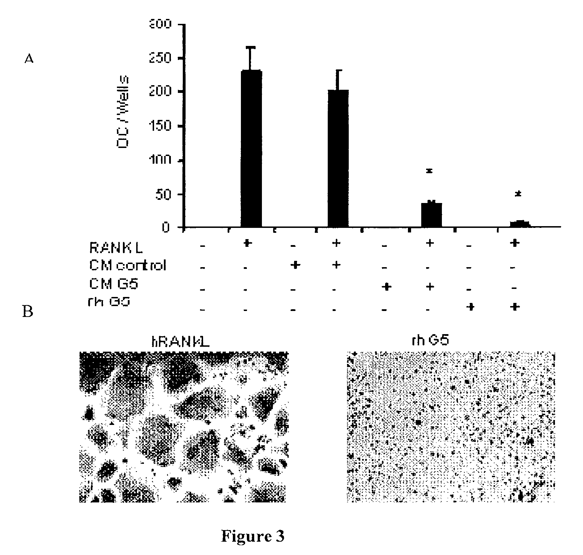

- la **Figure 3** montre l'inhibition de l'ostéoclastogénèse par HLA-G5. Des cellules monocytaires CD14^{pos} de sang périphérique d'origine humaine ont été induites à se différencier en ostéoclastes par l'ajout de M-CSF et du RANKL (RANKL, contrôle positif). Dans certains essais, il a été rajouté du milieu conditionné par la lignée M8 (cellules de mélanome HLA-G⁻) transfectée soit par un vecteur contenant un ADNc codant HLA-G5 (CM G5) soit par un vecteur contrôle ne contenant pas d'ADNc codant HLA-G5 (CM Control). Dans un autre essai, il a été ajouté la protéine HLA-G5 recombinante purifiée (rh G5). Après 20 jours de culture, les cellules adhérentes multinucléées TRAP^{pos} ont été comptées et rapportées en nombre d'ostéoclastes par puits (OC/wells) (voir le graphique A). Des ostéoclastes ont été détectés dans les conditions contrôles en présence du RANKL (photographie B « hRANKL ») contrairement aux conditions contenant le surnageant de la lignée M8-HLA-G5 ou contenant HLA-G5 recombinante (photographie **B** « rh G5 »). Le signe * indique que les différences sont significatives (p<0.01).

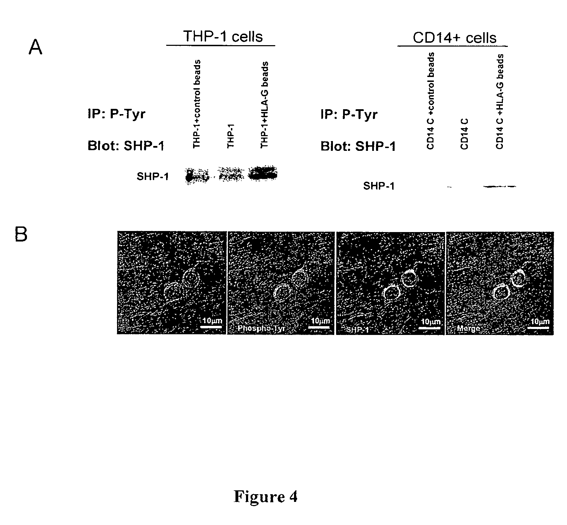

- la **Figure 4** montre la phosphorylation de SHP-1 dans des cellules monocytaires. **(A) :** des cellules monocytaires issues de la lignée THP-1 (« *THP-1 cells* ») ou CD14^{pos} de sang périphérique (« *CD14*+ *cells* » ou CD14 C) ont été incubées avec ou sans billes magnétiques recouvertes soit par un anticorps contrôle (« *control beads* ») soit par HLA-G5 (« *HLA-G beads* »). Les cellules ont ensuite été lysées et utilisées pour l'immunoprécipitation avec un anticorps anti-tyrosine phosphorylée (IP: P-Tyr). Après élution, les complexes retenus par la colonne ont été séparés par électrophorèse et transférés sur membrane PVDF. La protéine SHP-1 phosphorylée a ensuite été révélée par un anticorps anti-SHP-1. **(B) :** des cellules CD14^{pos} de sang périphérique ont été déposées sur une couche confluente de CSM puis fixées, perméabilisées et incubées avec des anticorps anti-tyrosine phosphorylée (2^{ème} photographie en partant de la gauche) et anti-SHP-1 (3^{ème} photographie en partant de la gauche). La 1^{ère} photographie en partant de la gauche représente les cellules en l'absence d'incubation avec les anticorps. La 4^{ème} photographie en partant de la gauche représente la fusion des 2^{ème} et 3^{ème} photographies. La préparation a ensuite été observée avec un microscope confocal en contraste de phase ; grossissement X630. En présence d'HLA-G, la phosphatase SHP-1 est recrutée ce qui se traduit pas une hyperphosphorylation de ses résidus tyrosine.

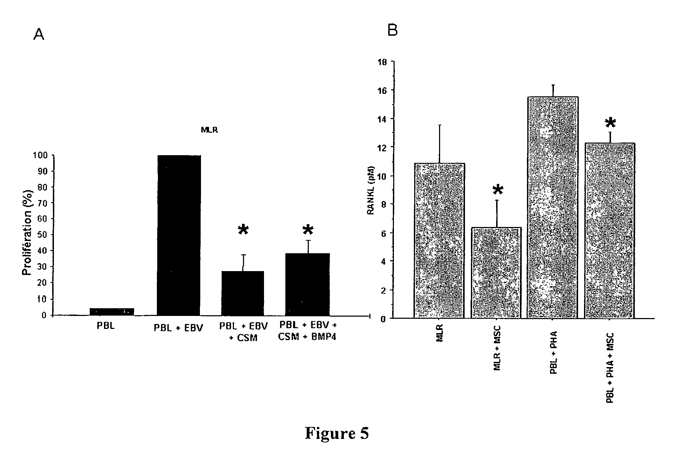

- la **Figure 5** montre la capacité des CSM à inhiber la prolifération lymphocytaire et à diminuer la sécrétion du RANKL par les lymphocytes activés. **(A) :** des cultures mixtes lymphocytaires (CML ou MLR) réalisées dans des plaques 96 puits et comprenant des lymphocytes de sang périphérique (PBL ou cellules répondantes) et des cellules B-EBV irradiées (cellules activatrices) ont été réalisées avec des CSMs en tierce partie (c'est-à-dire des cellules provenant d'un donneur allogénique) en présence ou non de BMP4 (20 ng/mL à J0 et J3). Après 6 jours de co-culture, la prolifération lymphocytaire est suivie par lecture de la fluorescence par un fluorimètre. Les différentes conditions testées ont été : PBL seuls (témoin négatif) ; PBL + B-EBV (témoin positif) ; PBL + B-EBV + CSM ; PBL + B-EBV + BMP4 ; PBL + B-EBV + CSM + BMP4. Nombre d'expériences effectuées : n = 3. **(B) :** des lymphocytes de sang périphérique (PBL) ont été activés soit par des cellules B-EBV (condition MLR) soit par un mitogène (phytohémagglutinine, PHA) (PBL + PHA) en présence ou non de MSC. Après 6 jours de culture, les surnageants ont été récupérés et le RANKL sécrété y a été dosé par ELISA (résultats rapportés en pM). Le signe * indique que les différences sont significatives (p<0.05). Nombre d'expériences effectuées : n=3.

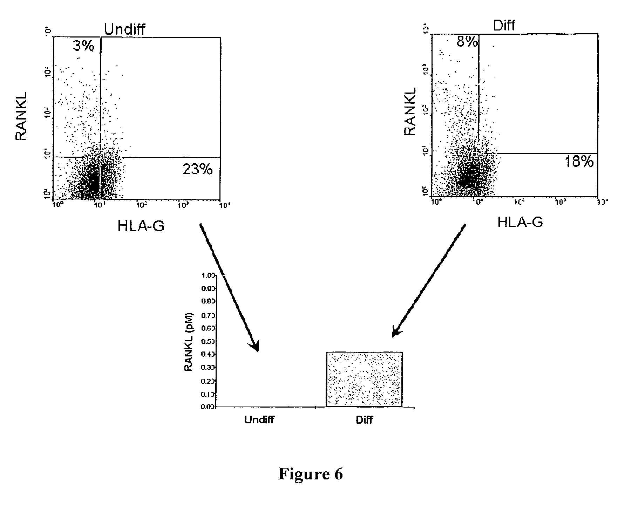

- la **Figure 6** montre que les CSM sécrétant RANKL sont HLA-G négatives. Des CSM ont été induites à se différencier (Diff) pour sécréter le RANKL ou n'ont pas été induites (Undiff). Les cellules ont ensuite été incubées avec des anticorps anti-RANKL et anti-HLA-G (87G) puis analysées par cytométrie en flux pour quantifier l'expression simultanée du RANKL et d'HLA-G (isoformes intracellulaires et membranaires). Les surnageants ont également été récupérés et la quantité du RANKL sécrétée a été dosée par ELISA (résultats rapportés en pM). Nombre d'expériences : n = 3.

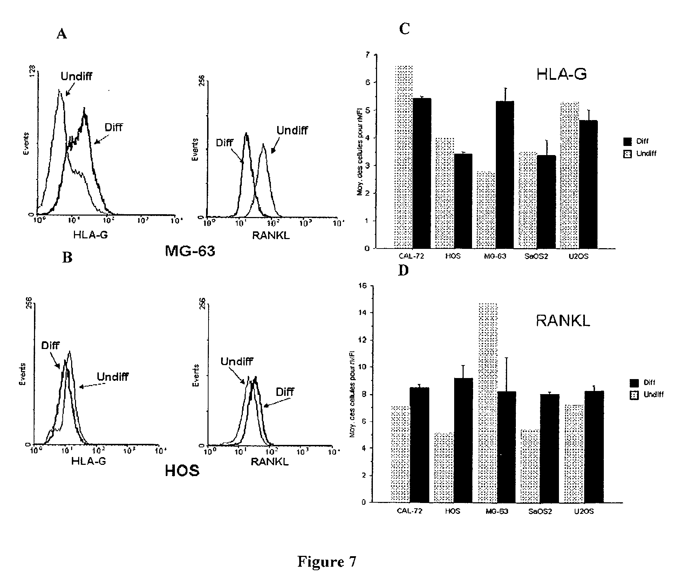

- la **Figure 7** montre les expressions inverses entre HLA-G et RANKL dans des lignées ostéoblastiques issues d'ostéosarcomes. Différentes lignées ostéoblastiques d'ostéosarcomes (CAL-72, HOS, MG-63, SaOs2 et U2OS) ont été induites à se différencier (Diff) ou non (Undiff). Ces lignées ont été clairement définies comme exprimant des marqueurs ostéoblastiques et forment de l'ostéoïde *in vivo.* Cependant, comme tous les ostéosarcomes, la population de cellule est hétérogène (Pautke *et al.,* 2004 ; Cleton-Jansen *et al.,* 2009). Après 6 jours de culture, les cellules ont été récupérées, fixées et perméabilisées. Après plusieurs lavages, les cellules ont été incubées avec des anticorps anti-RANKL et anti-HLA-G (87G) puis analysées par cytométrie en flux pour quantifier les expressions simultanées du RANKL et d'HLA-G (formes intracellulaires et membranaires). Les données sont rapportées sur les diagrammes **A** et **B** (moyenne des rapports de l'intensité de fluorescence ou rMFI) et les histogrammes **C** et **D.** Nombre d'expériences réalisées : n=4.

### EXEMPLE : INHIBITION IN VITRO DES OSTEOCLASTES PAR LES ISOFORMES D'HLA-G

### 1) Matériel et méthode

### a) Cellules souches mésenchymateuses (CMS)

Les cellules souches mésenchymateuses proviennent de ponctions de moelles osseuses de crête iliaque. Elles ont été obtenues chez des patients volontaires sains hospitalisés dans le service d'orthopédie-traumatologie du Centre Hospitalier Universitaire Trousseau (Tours, France) pour la mise en place d'une prothèse totale de hanche. Un consentement écrit du patient a été recueilli.

### b) Lignées d'ostéosarcomes

Les cinq lignées d'ostéosarcomes MG-63, Cal-72, HOS, U2OS et SaOS2, sont bien connues de l'Homme du métier. Elles ont été décrites par Billiau *et al.* (1977), Rochet *et al.* (1999), Fogh *et al.* (1975), Ponten et Saksela (1967) et Rhim *et al.* (1975).

### c) Cellules lymphocytaires de sang périphérique (PBL)

Du sang a été prélevé sur des personnes saines volontaires aux Etablissements Français du Sang (Tours, France). Les cellules mononucléées (CMN) de sang total (PBMC ou « *Peripheral Blood Mononuclear Cells* ») ont été obtenues après séparation sur gradient de Ficoll, et l'anneau de cellules a ensuite été mis en culture une nuit afin d'éliminer les monocytes adhérents. Après numération, les PBL (« *Peripheral Blood Lymphocytes* ») non adhérents inclus dans le surnageant ont été utilisés pour les co-cultures.

### d) Cellules B-EBV

Les cellules B-EBV sont des cellules B transformées par le virus d'Epstein Barr. Elles sont décrites par Sauce *et al.* (2004).

### e) Cellules CD14^{pos}

Les cellules CD14^{pos} ont été sélectionnées à partir de PBMC en utilisant le kit de purification de Miltenyi CD 14 microbeads kit (Myltenyi ; Bergisch Gladbach, Allemagne) selon les recommandations du fournisseur.

### f) Culture et sélection cellulaire

Après transport conditionné en acide citrique dextrose (ACD), les prélèvements de moelles osseuses ont été directement placés dans des flacons de culture (BD Biosciences, VWR, Strasbourg, France). Le milieu de culture a été changé toutes les 48 heures. Les cellules non adhérentes ont été éliminées. Après 7 à 15 jours de culture, les cellules adhérentes, arrivant à confluence, ont été détachées à l'aide de la trypsine à 0,5% (v/v) (In Vitrogen, Fischer Bio Block Scientific, Illkirch, France), puis mises en suspension pour être caractérisées ou réensemencées à 200 000 cellules/cm² pour une nouvelle prolifération. Les cellules ont été maintenues dans des incubateurs en atmosphère humide avec 5% de CO2 et à une température de 37°C.

### g) Milieux de culture

### - Milieux de prolifération

Le milieu de prolifération utilisé est composé de Minimal Essential Medium alpha (αMEM) (In Vitrogen), de 10% de sérum de veau foetal (SVF) (Perbio Hyclone, Logan, Etats-Unis), de 1% de pénicilline-streptomycine et L-glutamine (In Vitrogen) et de fungizone (Bristol Myers Squibb, Rueil Malmaison, France). Les lignées ostéosarcomateuses ont été cultivées dans ce même milieu.

### - Milieu de différenciation ostéocytaire

Ce milieu est composé de αMEM, de 0,05.10⁻³ M acide ascorbique, de 10.10⁻³ M P-glycérophosphate (Sigma Aldrich, Lyon, France) et de 50ng/ml de BMP4 (R&D Systems, Minneapolis, MN). Il permet la différenciation ostéocytaire après 14 jours de culture.

### - Milieu d'induction ostéoclastique

Des cellules CD14^{pos} ont été cultivées dans des plaques 96 puits contenant du milieu complémenté par M-CSF 25 ng/ml et RANKL 30 ng/ml (R&D Systems) pendant 15 jours. La quantification du nombre de cellules multinucléées adhérentes au fond des puits et positives pour TRAP à été effectuée à 20 jours. L'activité TRAP a été mise en évidence à l'aide du kit TRAP Assay (Sigma). Du milieu conditionné par les lignées M8-HLA-G5 ou M8-vecteur contrôle (Naji *et al.,* 2007a et 2007b) ou de l'HLA-G5 humain recombinant (rhHLA-G5 ; Biovendor, Modrice, République Tchèque) ont pu être rajoutés au milieu d'induction ostéoclastique.

### - Milieu de différenciation de cellules dendritiques

Les cellules CD14^{pos} issues de PBMC ont été cultivées dans un milieu contenant 50ng/ml de rhGM-CSF et 20ng/ml rhIL-4 (R&D Systems) pendant 6 jours. Cela a constitué le milieu de contrôle positif (MONO). A des fractions de milieu de cellules CD14^{pos}, il a été rajouté soit du surnageant de CSM avec un anticorps contrôle soit avec l'anticorps anti-HLA-G bloquant (87G) (Exbio, Vestec, République Tchèque). Après 6 jours de culture, les cellules ont été récoltées et analysées au cytomètre en flux.

### h) Cytométrie en flux

Pour mettre en évidence un antigène membranaire, 200 000 cellules vivantes sont marquées par un anticorps monoclonal spécifique couplé à un fluorochrome. Pour mettre en évidence un antigène intracellulaire, les cellules sont fixées et perméabilisées à l'aide du kit Cytofix/Cytoperm™ (Becton Dickinson, Erembodegem, Belgique) en suivant les recommandations du fournisseur. Le marquage a été obtenu après 30 minutes d'incubation à 4°C à l'obscurité. Les cellules ont ensuite été rincées avec du Phosphate Buffered Saline (PBS), puis fixées au CellFIX® (Becton Dickinson). Les cellules ont alors été passées au cytomètre en flux (FACS Calibur®, Becton Dickinson), équipé d'un laser Argon, émettant une longueur d'onde de 488 nm. Les données ont été analysées à l'aide du logiciel CellQuest 3.1® (Becton Dickinson). Les résultats sont exprimés en rapport de moyenne d'intensité de fluorescence (RMFI) du signal détecté par rapport à celui du bruit de fond. Pour détecter HLA-G, les anticorps utilisés étaient ceux produits par le clone 87G conjugué à l'Alexa488 (reconnaissant les isoformes HLA-G1 et -G5), le clone MEM/G9 conjugué à l'APC (reconnaissant aussi les isoformes HLA-G1 et -G5) et le clone 5A6G7 conjugué à l'Alexa488 (reconnaissant les isoformes HLA-G5 et -G6) (Exbio). Pour détecter RANKL, l'anticorps utilisé était celui produit par le clone MIH24 (eBioscience ; San Diego, CA). Pour détecter ILT2, l'anticorps utilisé était celui produit par le clone VMP55 (Santa Cruz Biotechnology, Santa Cruz, CA). Pour détecter ILT4, l'anticorps utilisé était celui produit par le clone 42D1 (Santa Cruz Biotechnology). Pour détecter CD 14, l'anticorps utilisé était celui produit par le clone 134620 (R&D Systems). Pour détecter CD1a l'anticorps utilisé était celui produit par le clone HI149 (Becton Dickinson).

### i) Microscopie confocale

Les cellules à observer ont été ensemencées dans des chambres puits de 1 cm² de surface sur lames (Labteks®, Nunc International, Rochester, New York, USA) à 10 000 cellules/puits. Après 48 heures de culture, elles ont été fixées 10 minutes avec du paraformaldéhyde (Sigma) à 4%. Une étape de perméabilisation avec une solution de PBS, 0,5% de SVF et 0,1% de tritonX100 (BioRad, Hercules, California, USA), pendant 5 minutes à température ambiante, a été nécessaire pour identifier les protéines intracellulaires. Les cellules ont ensuite été incubées successivement avec les anticorps primaires anti-tyrosine phosphorylée (4G10, Millipore, Billerica, MA) ou anti-SHP-1 (R&D Systems), pendant 1 heure à 4°C et les anticorps secondaires correspondants conjugués à un fluorochrome de type Alexa 488 ou 594 (In Vitrogen). Après rinçage, un milieu de montage (Vector Cliniscience, Montrouge, France) a été ajouté. Des puits contenant des anticorps contrôles ont servi de contrôles négatifs. Les lames ont été observées au microscope confocal (Olympus, FluoviewTM FV1000 ; Hamburg, Allemagne).

### j) Culture mixte lymphocytaire (CML ou MLR) :

Les cultures mixtes lymphocytaires ont été réalisées sur une plaque 96 puits. Les PBL (cellules répondantes) ont été ensemencées à raison de 10⁵ cellules. Les B-EBV (cellules stimulatrices) ont été irradiées (75 Gy) et utilisées à 5.10⁴ cellules. Les CSMs ont été co-cultivées à raison de 5.10⁴ cellules. La BMP4 a été utilisée à une concentration de 20 ng/ml à J0 et J3. Après 6 jours de co-culture, l'incorporation de BrdU (5-bromo-2'dioxyuridine) a été suivie grâce à un anticorps anti-Brdu couplé à l'Europium (Perkin Elmer, Waltham, MA). La quantité d'europium a été mesurée à l'aide du kit *Delfia proliferation kit* (Perkin Elmer). La fluorescence de l'europium observée par DELFIA (*Dissociation-Enhanced Lanthanide Fluorescent Immunoassay*) est proportionnelle à l'ADN synthétisé et donc à la prolifération lymphocytaire. Dans certaines expériences, la phytohémagglutinine (PHA) (R&D Systems) à 10 µg/ml a été utilisée pour activer les PBL.

### k) Immunoprécipitation

Le kit µMACS™ Protein A/G Microbeads (Miltenyi) a été utilisé pour effectuer les immunoprécépitations. Les échantillons de cellules ont été traités selon les recommandations du fournisseur. Après élution de la fraction non retenue, les échantillons protéiques immunoprécipités ont été analysés par *Western blotting.*

### 1) Western blotting

Les cellules (1.10⁶ cellules) ont été lysées dans un tampon spécifique (Lysis Buffer) : 1% de Sodium Dodecyle Sulfate (SDS) (Sigma), 1 mM orthovanadate de sodium (Sigma), 10mM Tris pH 7.4 (Sigma) complété d'une solution d'antiprotéases (Roche Diagnostic, Mannheim, Allemagne). Les échantillons ont été dosés par le mini Kit BCA Pierce (Pierce, New York, USA). La concentration a été évaluée par spectrophotomètrie à 562 nm. Les échantillons destinés à être analysées par *Western blotting,* ont été additionnés à du tampon d'échantillon (sample buffered) 1 X, composé de 0.0625 M TRIS, 2% SDS, 20% Glycérol, 0,01% Bleu de Bromophénol, 0,2% β-Mercaptoéthanol, et placés 3 minutes à 95 °C. Un gel a été réalisé sur un kit de montage (Bio-rad, Californie, USA). Un gel de séparation à 10% d'acrylamide (Acrylamide bisacrylamide 37,5/1 à 40%) composé de tampon de séparation 1,5 mM Tris HCl pH 8,8, 10% acrylamide, 0,012% TEMED (N,N,N,N-Tétramethyl-Ethylènediamine ; Sigma) et 0,05% d'APS (persulfate d'ammonium ; Sigma) a été coulé et un deuxième gel de concentration à 4% d'acrylamide composé de tampon de concentration (0,5 mM Tris HCl pH 6,8, 4% acrylamide, 0,1% SDS, 0,03% TEMED et 0,05% APS), a été déposé dessus. Après polymérisation les échantillons ont été déposés à raison de 10 à 20 µg par puits ainsi qu'un marqueur de poids moléculaire (Précision Plus Protein Standards, Bio-rad). La migration s'est effectuée successivement pendant 15 minutes à 70 mV, 5 minutes à 100 mV et à 120 mV jusqu'à la fin de la migration dans du tampon de migration (25 mM Tris, 0,19 M glycine et 1% SDS). Le matériel de transfert (Mini protean II ; Bio-rad) ainsi que le gel d'acrylamide ont été équilibrés dans du tampon de transfert composé de 2% de tampon de migration préparé précédemment et 5% méthanol. Le transfert a été effectué sur une membrane de nitrocellulose de 0,45 µm (Protran BA85 cellulosenitrate, Schleicher & Schull, Dassel, Allemagne) préalablement hydratée pendant 2 heures, à 250 mA dans du tampon de transfert. Après le transfert, les membranes ont été saturées au PBS Tween 0,1% (Sigma), 5% de lait (écrémé, pauvre en calcium) pendant 1 h à température ambiante. Les membranes ont été incubées avec les anticorps primaires anti-SHP-1 (R&D Systems) dilués dans du PBS Tween 0,1%, 5% lait, toute la nuit à 4°C. Les anticorps de lapin anti-chèvre couplés à la HRP (Horse Radish Peroxydase) (Jackson ImmunoResearch, West Grove, Pennsylvanie, USA) ont été utilisés comme anticorps secondaires. L'incubation a eu lieu à température ambiante pendant 2 heures. Les membranes ont été incubées avec le substrat de la HRP (Opti-4CN substrat kit ; Bio-Rad) jusqu'à l'obtention de bandes colorées.

### m) Analyses par ELISA (Enzyme Linked ImmunoSorbent Assay)

Les concentrations d'HLA-G et du RANKL sécrétées dans les surnageants des différentes cultures cellulaires réalisées ont été mesurées. Les kits ELISA pour doser HLA-G5 (Exbio) et RANKL (Biovendor) ont été utilisés selon les recommandations du fournisseur.

### 2) Résultats

Il a été rapporté dans la littérature que les cellules ostéoclastiques pouvaient être obtenues à partir de monocytes de sang périphérique ou de cellules dendritiques. Il est aussi connu que les cellules monocytaires expriment à leur surface les récepteurs d'HLA-G ILT2 et ILT4.

Il a été étudié si l'expression des récepteurs ILT2 et ILT4 d'HLA-G par les cellules monocytaires était conservée lorsque ces cellules sont co-cultivées avec des CSM de moelles osseuses et si ces dernières pouvaient les exprimer.

Après environ 6 jours de cultures, les données de cytométrie en flux (voir Figure 1) ont montré que seules les cellules monocytaires CD14^{pos} exprimaient ILT2 et ILT4 et que ces expressions étaient bien maintenues dans le temps.

Il a ensuite été recherché si HLA-G pouvait inhiber la différenciation ostéoclastique lors de la différenciation des monocytes en cellules dendritiques ou lors de l'ostéoclastogénèse à partir de monocytes.

Des monocytes de sang périphérique CD14^{pos}/CD1a^{neg} ont été stimulés avec du GM-CSF et de l'IL4 pour obtenir des cellules dendritiques CD14^{neg}/CD1a^{pos}. Les ajouts de surnageants de culture de CSM ont inhibé la différenciation des cellules (la majorité était CD14^{pos}/CD1a^{neg}) contrairement aux conditions contrôles où la majorité des cellules sont devenues CD14^{neg} dont une fraction exprimait le CD1a. L'ajout de l'anticorps bloquant anti-HLA-G (87G) a levé partiellement l'inhibition observée avec les CSM puisqu'il a été détecté des cellules exprimant le CD1a (voir Figure 2).

Des monocytes CD14^{pos} ont ensuite été induits à se différencier directement en ostéoclastes après ajout de M-CSF et du RANKL (conditions contrôles positifs). Dans certaines conditions il a été ajouté i) des surnageants de milieux conditionnés par une lignée M8 transfectée ou non par le cDNA codant HLA-G5 (G5 CM et CM Ctrl respectivement) ou ii) de l'HLA-G5 humain recombinant (rh G5).

Alors que dans les contrôles positifs et dans les conditions CM Ctrl, les ostéoclastes ont été aisément observés, il y en a été peu observés dans les conditions G5 CM (voir Figure 3). Dans les conditions où l'HLA-G5 recombinant a été utilisé, il a été rarement détecté des ostéoclastes. HLA-G5 est donc capable d'inhiber la génération d'ostéoclastes à partir de monocytes.

Il est connu que les récepteurs ILT2 et ILT4 possèdent des motifs ITIM dans leur partie intracytoplasmique. Ces motifs sont capables d'activer des phosphatases de type SHP-1 au niveau des tyrosines quand ces récepteurs sont liés à leur ligand (Barrow et Trowsdale, 2006).

Il a donc été évalué l'activation de SHP-1 dans les cellules monocytaires CD14^{pos} normales ou de lignée THP-1. Ces cellules ont été incubées avec des billes recouvertes d'HLA-G ou des billes seules en tant que contrôle négatif. Les billes ont été obtenues selon la méthode décrite par Naji *et al.* (2007a).

Les résultats représentés à la Figure 4 montrent une nette augmentation de SHP-1 phosphorylée quand les cellules sont mises en contact avec des billes HLA-G^{pos} et ce quelque soit le type de cellules monocytaires (THP-1 ou les cellules CD14^{pos}) (voir Figure 4A). Par ailleurs, quand des monocytes CD14^{pos} sont co-cultivés avec des CSM, il a été observé une colocalisation de l'expression de tyrosines phosphorylées et de SHP-1 (voir Figure 4B). Ces données montrent qu'HLA-G induit l'activation de protéines inhibitrices de la différenciation des cellules monocytaires.

L'ostéoclastogénèse est dépendante de la molécule RANKL. Cette dernière peut être sécrétée principalement par des cellules lymphocytaires et par les ostéoblastes eux mêmes. Il a donc été cherché s'il existait un lien entre l'expression d'HLA-G et du RANKL.

La Figure 5A montre que les CSM sont capables d'inhiber la prolifération de lymphocytes dans des conditions de culture mixte lymphocytaire. L'ajout de BMP4 n'a pas augmenté cet effet.

Il a ensuite été quantifié la sécrétion du RANKL dans les surnageants de ces co-cultures. Il a été constaté que dans chaque condition contenant les CSM, il y avait une diminution significative de la sécrétion du RANKL et ce même quand les lymphocytes ont été activés par du PHA (voir Figure 5B). Ces résultats suggèrent que l'inhibition de l'activation des lymphocytes par les CSM diminue aussi la sécrétion du RANKL.

Il a été précédemment montré par certains des inventeurs que cette inhibition était en partie due à HLA-G et que l'interaction entre lymphocytes allogéniques et CSM induisait le relargage d'HLA-G dans le milieu de culture (Selmani *et al.,* 2008).

Il a donc été vérifié si une relation inverse existait entre l'expression d'HLA-G et du RANKL. Des CSM ont été induits pour produire le RANKL par induction de la différenciation ostéoblastique. En effet, il est connu que les ostéoblastes produisent le RANKL (Lorenzo *et al.,* 2008). La différenciation ostéoblastique a diminué l'expression d'HLA-G1 et d'HLA-G5 au niveau des CSM et augmenté de manière concomitante l'expression du RANKL (voir Figure 6). Ce résultat a été confirmé par la technique d'ELISA puisqu'il a été possible de détecter RANKL dans les surnageants des cultures induites (Diff) contrairement à celles qui ne l'ont pas été (Undiff).

Il a ensuite été évalué cette relation dans des cellules ostéoblastiques issues de lignées d'ostéosarcomes. D'une manière remarquable, il a aussi été observé une relation inverse entre l'expression du RANKL et d'HLA-G. Pour les lignées CAL-72, HOS, SaOs2 et U2OS l'induction de la différenciation ostéoblastique a diminué l'expression d'HLA-G1 et d'HLA-G5 et concomitamment augmenté l'expression du RANKL. Pour la lignée MG-63 la différenciation a augmenté l'expression d'HLA-G et diminué celle du RANKL (voir Figure 7).

Il ressort de ces résultats qu'HLA-G exprimée par les CSM ou les cellules ostéoblastiques (normales ou tumorales) inhibe la résorption osseuse de deux manières : soit directement en supprimant l'ostéoclastogénèse, soit indirectement en inhibant l'activation des cellules sécrétant RANKL. Par ailleurs, il existe une relation inverse d'expression entre HLA-G et RANKL dans les cellules ostéoblastiques.

### Bibliographie

- Barrow AD et Trowsdale J., Eur J Immunol. 2006; 36: 1646-1653
- Billiau A, et al., Antimicrob Agents Chemother. 1977; 12: 11-15
- Carosella ED, et al., Trends Immunol. 2008a; 29: 125-132
- Carosella ED, et al., Blood. 2008b; 111: 4862-4870
- Cleton-Jansen AM, et al., Br J Cancer. 2009; 101: 1909-1918
- Cohen MM, Jr., Am J Med Genet A. 2006; 140: 2646-2706
- Deschaseaux F, et al., Trends Mol Med. 2009a; 15: 417-429
- Deschaseaux F, et al., J Cell Mol Med. 2009b; 14: 103-115
- Duplomb L, et al., Stem Cells. 2007; 25: 544-552
- Ellis S., Am JReprod Immunol. 1990; 23: 84-86
- Fogh, J., et al., in J.Fogh (ed.), Human Tumor Cell Lines in vitro. New York: Plenum Press 1975; 115-159.
- Geraghty DE, et al., Proc Natl Acad Sci U S A. 1987; 84: 9145-9149
- Hackmon R, et al., Fetal Diagn Ther. 2004; 19: 404-409
- Ishitani A et Geraghty DE., Proc Natl Acad Sci U S A. 1992; 89: 3947-3951
- Kirszenbaum M, et al., Proc Natl Acad Sci USA. 1994; 91: 4209-4213
- Kirszenbaum M, et al., Hum Immunol. 1995; 43: 237-241
- Le Maoult J, et al., FASEB J. 2005; 19: 662-664
- Lorenzo J, et al., Endocr Rev. 2008; 29: 403-440
- Moreau P, et al., Hum Immunol. 1995; 43: 231-236
- Naji A, et al., Blood. 2007a; 110: 3936-3948
- Naji A, et al., Hum Immunol. 2007b; 68: 233-239
- Pautke C, et al., Anticancer Res. 2004; 24: 3743-3748
- Ponten J et Saksela E., Int J Cancer. 1967; 2: 434-447
- Rebmann V, et al., Tissue Antigens. 1999; 53: 14-22
- Rhim JS, et al., Nature. 1975; 256: 751-753
- Rochet N, et al., Int J Cancer. 1999; 82: 282-285
- Rouas-Freiss N, et al., Transplantation. 2007; 84: S21-25
- Sauce D, et al., Gene Ther. 2004; 11: 1019-1022
- Selmani Z, et al., Stem Cells. 2008; 26: 212-222

## Revendications

1. Isoforme d'HLA-G isolée pour utilisation comme médicament pour le traitement ou la prévention d'une maladie dans laquelle on observe une résorption osseuse.

2. Isoforme pour l'utilisation selon la revendication 1, **caractérisée en ce que** ladite maladie est choisie dans le groupe constitué par l'ostéoporose, l'ostéopénie, les fractures osseuses, la maladie de Paget et les tumeurs ostéolytiques.

3. Isoforme pour l'utilisation selon la revendication 1 ou la revendication 2, **caractérisée en ce qu'**elle est une isoforme soluble.

4. Isoforme pour l'utilisation selon la revendication 1 ou la revendication 2, **caractérisée en ce qu'**elle est choisie dans le groupe constitué par HLA-G1 et HLA-G5, de préférence HLA-G5.

5. Isoforme pour l'utilisation selon l'une quelconque des revendications 1 à 4, **caractérisée en ce qu'**elle est sous une forme monomérique.

6. Isoforme pour l'utilisation selon l'une quelconque des revendications 1 à 4, **caractérisée en ce qu'**elle est sous une forme multimérique.

7. Composition pharmaceutique comprenant une isoforme isolée d'HLA-G et au moins un véhicule pharmaceutiquement acceptable pour utilisation comme médicament pour le traitement ou la prévention d'une maladie dans laquelle on observe une résorption osseuse.

## Patentansprüche

1. Isoliertes HLA-G-Isoform für die Verwendung als Arzneimittel zur Behandlung oder zur Prävention einer Krankheit, bei der eine Knochenresorption beobachtet wird.

2. Isoform für die Verwendung nach Anspruch 1, **dadurch gekennzeichnet, dass** die Krankheit aus der Gruppe ausgewählt ist, die von der Osteoporose, der Osteopenie, den Knochenfrakturen, dem Morbus Paget und den osteolytischen Tumoren gebildet ist.

3. Isoform für die Verwendung nach Anspruch 1 oder Anspruch 2, **dadurch gekennzeichnet, dass** es ein lösliches Isoform ist.

4. Isoform für die Verwendung nach Anspruch 1 oder Anspruch 2, **dadurch gekennzeichnet, dass** es aus der Gruppe ausgewählt ist, die von HLA-G1 und HLA-G5, vorzugsweise HLA-G5, gebildet ist.

5. Isoform für die Verwendung nach einem der Ansprüche 1 bis 4, **dadurch gekennzeichnet, dass** es in einer Monomerform vorliegt.

6. Isoform für die Verwendung nach einem der Ansprüche 1 bis 4, **dadurch gekennzeichnet, dass** es in einer Multimerform vorliegt.

7. Pharmazeutische Zusammensetzung, umfassend ein isoliertes HLA-G-Isoform und mindestens ein pharmazeutisch akzeptables Vehikel für die Verwendung als Arzneimittel zur Behandlung oder zur Prävention einer Krankheit, bei der eine Knochenresorption beobachtet wird.

## Claims

1. An isolated isoform of HLA-G for use as a medicament for the treatment or prevention of a disease in which bone resorption is observed.

2. The isoform for use as claimed in claim 1, **characterized in that** said disease is chosen from the group consisting of osteoporosis, osteopenia, bone fractures, Paget's disease and osteolytic tumors.

3. The isoform for use as claimed in claim 1 or claim 2, **characterized in that** it is a soluble isoform.

4. The isoform for use as claimed in claim 1 or claim 2, **characterized in that** it is chosen from the group consisting of HLA-G1 and HLA-G5, preferably HLA-G5.

5. The isoform for use as claimed in any one of claims 1 to 4, **characterized in that** it is in a monomeric form.

6. The isoform for use as claimed in any one of claims 1 to 4, **characterized in that** it is in a multimeric form.

7. A pharmaceutical composition comprising an isolated isoform of HLA-G and at least one pharmaceutically acceptable vehicle for use as a medicament for the treatment or prevention of a disease in which bone resorption is observed.
